# EUROPEAN PATENT APPLICATION

(11) **EP 4 467 148 A1**
(43) Date of publication of application: **27.11.2024**
(21) Application number: 23743450.1
(22) Date of filing: 18.01.2023
(51) Int. Cl.: A61K 35/74, A61K 35/745, A61P 1/16, A23L 33/135, A23K 10/16

(54) **COMPOSITION FOR PREVENTION, AMELIORATION OR TREATMENT OF NON-ALCOHOLIC FATTY LIVER DISEASE, COMPRISING EXTRACELLULAR VESICLES DERIVED FROM ROSEBURIA SPP. OR BIFIDOBACTERIUM SPP**

(30) Priority: 20.01.2022 KR 20220008555; 17.01.2023 KR 20230006808
(71) Applicant: Korea Institute of Science and Technology, Seoul 02792 (KR)
(72) Inventor: CHOI, Ki Young, Gangneung-si, Gangwon-do 25451 (KR); HAN, Hwa Seung, Gangneung-si, Gangwon-do 25451 (KR); CHA, Kwang-Hyun, Gangneung-si, Gangwon-do 25451 (KR); SONG, Dae-geun, Gangneung-si, Gangwon-do 25451 (KR); PARK, Jin Soo, Gangneung-si, Gangwon-do 25451 (KR); LEE, Choong-Gu, Gangneung-si, Gangwon-do 25451 (KR); KIM, Myung Suk, Gangneung-si, Gangwon-do 25451 (KR)
(74) Representative: Dehns
(86) International application number: PCT/KR2023/000839
(87) International publication number: WO 2023/140603

(57) **Abstract**

The present invention relates to a composition for the prevention, amelioration or treatment of non-alcoholic fatty liver disease (NAFLD), comprising a *Roseburia* spp. strain, a *Bifidobacterium* spp. strain, or extracellular vesicles (EVs) derived therefrom. The *Roseburia* spp. strain, the *Bifidobacterium* spp. strain, or the EVs derived therefrom, according to the present invention, can restore liver function, ameliorate liver steatosis and inflammation, and suppress liver fibrosis, and thus can be provided as a composition for the prevention, amelioration or treatment of NAFLD.

## Description

### [Technical Field]

The present invention relates to a composition for prevention, amelioration, or treatment of non-alcoholic fatty liver disease (NAFLD), including a *Roseburia* spp. strain, a *Bifidobacterium* spp. strain, or extracellular vesicles (EVs) derived therefrom.

The present invention claims priority based on Korean Patent Application No. 10-2022-0008555 filed on January 20, 2022 and Korean Patent Application No. 10-2023-0006808 filed on January 17, 2023, and all contents disclosed in the specification and drawings of the above applications are incorporated in the present application by reference.

### [Background Art]

The liver is an organ that plays a central role in metabolism, and when liver function is abnormal, problems occur in the overall nutrient metabolism of an individual. Among liver diseases, fatty liver refers to a phenomenon in which neutral fat that is not present in normal cells is abnormally deposited in liver cells. A normal liver is composed of about 5% adipose tissue, which consists of neutral fat, fatty acids, phospholipids, cholesterol, and cholesterol esters, but when fatty liver begins to develop, most fat components are replaced by neutral fat, and when the amount of neutral fat is more than or equal to 5% of the total liver weight, fatty liver is diagnosed. When fatty liver worsens and the fat mass in the liver cells increases, important cell components such as the nucleus are pushed to one side, and the liver cells expanded due to the accumulated fat press the surrounding microvessels and lymph nodes, resulting in the circulation of blood and lymph being disrupted within the liver. When this happens, the liver cells may not be properly supplied with oxygen and nutrients, which leads to impaired liver function.

Fatty liver may be divided into alcoholic fatty liver caused by excessive drinking, and non-alcoholic fatty liver. Non-alcoholic fatty liver disease (NAFLD) is a metabolic liver disease resulting from metabolic dysregulation, which is caused by fat accumulation in the liver that is not related to alcohol consumption. NAFLD encompasses a variety of diseases ranging from simple steatosis in the liver to steatohepatitis with inflammation, or cirrhosis (Brunt EM, 2001), and may be caused by fat deposition in liver cells due to obesity or insulin resistance, diabetes, and genetic causes. In particular, unlike simple fatty liver, which has a good clinical prognosis, non-alcoholic steatohepatitis (NASH) is a progressive liver disease with a high risk of progressing to cirrhosis and liver cancer, so timely and appropriate treatment is required (Angulo P., Hepatology, 2010;51:373-375).

As the number of patients with NAFLD increases, the need for drug development for the prevention, amelioration, and treatment of the disease is increasing. However, there is no drug that has been officially approved as a drug treatment for NAFLD yet, and the development of a drug that can fundamentally treat NAFLD is required.

### [Disclosure]

### [Technical Problem]

As a result of diligent efforts to find a drug having a better effect on NAFLD than conventional therapeutic agents, the inventors of the present invention have completed the present invention by confirming that *Roseburia* spp. strains, *Bifidobacterium* spp. strains, or extracellular vesicles (EVs) derived therefrom exhibit excellent prevention, amelioration, and treatment effects on NAFLD.

Therefore, an object of the present invention is to provide a pharmaceutical composition for preventing or treating NAFLD, including one or more selected from the group consisting of EVs derived from a *Roseburia* spp. strain and EVs derived from a *Bifidobacterium* spp. strain as an active ingredient.

Another object of the present invention is to provide a kit for preventing or treating NAFLD, including one or more selected from the group consisting of EVs derived from a *Roseburia* spp. strain and EVs derived from a *Bifidobacterium* spp. strain.

Still another object of the present invention is to provide a method of preventing or treating NAFLD, including administering one or more selected from the group consisting of EVs derived from a *Roseburia* spp. strain and EVs derived from a *Bifidobacterium* spp. strain to a subject in need thereof.

Yet another object of the present invention is to provide a food composition for preventing or ameliorating NAFLD, including one or more selected from the group consisting of EVs derived from a *Roseburia* spp. strain and EVs derived from a *Bifidobacterium* spp. strain.

Yet another object of the present invention is to provide a feed composition for preventing or ameliorating NAFLD, including one or more selected from the group consisting of EVs derived from a *Roseburia* spp. strain and EVs derived from a *Bifidobacterium* spp. strain.

However, the technical problems to be solved by the present invention are not limited to the above-mentioned problems, and other problems not mentioned will be clearly understood by those skilled in the art from the description below.

### [Technical Solution]

The present invention provides a pharmaceutical composition for preventing or treating NAFLD, including one or more selected from the group consisting of EVs derived from a *Roseburia* spp. strain and EVs derived from a *Bifidobacterium* spp. strain as an active ingredient.

In addition, the present invention provides a food composition for preventing or ameliorating NAFLD, including one or more selected from the group consisting of EVs derived from a *Roseburia* spp. strain and EVs derived from a *Bifidobacterium* spp. strain as an active ingredient.

In addition, the present invention provides a feed composition for preventing or ameliorating NAFLD, including one or more selected from the group consisting of EVs derived from a *Roseburia* spp. strain and EVs derived from a *Bifidobacterium* spp. strain as an active ingredient.

In addition, the present invention provides a kit for preventing, ameliorating, or treating NAFLD, including one or more selected from the group consisting of EVs derived from a *Roseburia* spp. strain and EVs derived from a *Bifidobacterium* spp. strain.

Furthermore, the present invention provides a method of preventing or treating NAFLD, including administering one or more selected from the group consisting of EVs derived from a *Roseburia* spp. strain and EVs derived from a *Bifidobacterium* spp. strain to a subject in need thereof.

Furthermore, the present invention provides a use of one or more selected from the group consisting of EVs derived from a *Roseburia* spp. strain and EVs derived from a *Bifidobacterium* spp. strain for preventing or treating NAFLD.

Furthermore, the present invention provides a use of one or more selected from the group consisting of EVs derived from a *Roseburia* spp. strain and EVs derived from a *Bifidobacterium* spp. strain for preparing a therapeutic agent for NAFLD.

In one embodiment of the present invention, the average diameter of the EVs may be 30 nm to 200 nm, but is not limited thereto.

In another embodiment of the present invention the EVs derived from a *Roseburia* spp. strain may be naturally or artificially secreted from a *Roseburia* spp. strain; and

In still another embodiment of the present invention, the EVs derived from a *Bifidobacterium* spp. strain may be naturally or artificially secreted from a *Bifidobacterium* spp. strain, but are not limited thereto.

In yet another embodiment of the present invention, the *Roseburia* spp. strain may be one or more selected from the group consisting of *Roseburia intestinalis, Roseburia faecis, Roseburia hominis,* and *Roseburia inulinivorans,* but is not limited thereto.

In yet another embodiment of the present invention, the *Bifidobacterium* spp. strain may be one or more selected from the group consisting of *Bifidobacterium longum, Bifidobacterium bifidum, Bifidobacterium breve, Bifidobacterium lactis,* and *Bifidobacterium adolescentis,* but is not limited thereto.

In yet another embodiment of the present invention, the NAFLD may be one or more selected from the group consisting of steatosis, fibrosis, non-alcoholic steatohepatitis, non-alcoholic fatty liver (NAFL), and cirrhosis, but is not limited thereto.

In yet another embodiment of the present invention, the pharmaceutical composition according to the present invention may have one or more effects selected from the group consisting of:
i) reducing serum aspartate aminotransferase (AST) levels or activity;
ii) reducing serum alanine aminotransferase (ALT) levels or activity;
iii) reducing serum total bilirubin (T-BIL) levels;
iv) reducing hepatic steatosis;
v) reducing inflammatory cell infiltration;
vi) reducing the number of inflammatory foci; and
vii) reducing the number of myofibroblasts, but is not limited thereto

In yet another embodiment of the present invention, the food composition may be a health functional food composition, but is not limited thereto.

### [Advantageous Effects]

It was confirmed that the EVs derived from a *Roseburia* spp. strain or a *Bifidobacterium* spp. strain of the present invention restore liver function, ameliorate steatosis by reducing fat accumulation in the liver, and exhibit anti-inflammatory effects such as inhibiting the infiltration of inflammatory cells into liver tissue, as well as inhibiting liver fibrosis by reducing the expression of myofibroblasts in an NAFLD animal model. Furthermore, it was also confirmed that all EVs derived from the congeneric strains belonging to *Roseburia* spp. and *Bifidobacterium* spp. strains exert strong anti-inflammatory effects. As such, the EVs derived from *Roseburia* spp. strains and EVs derived from *Bifidobacterium* spp. strains can ameliorate liver damage and dysfunction due to fatty liver and exert strong anti-inflammatory and anti-fibrotic effects, and thus can be used as a composition for preventing, ameliorating, and/or treating NAFLD.

### [Description of Drawings]

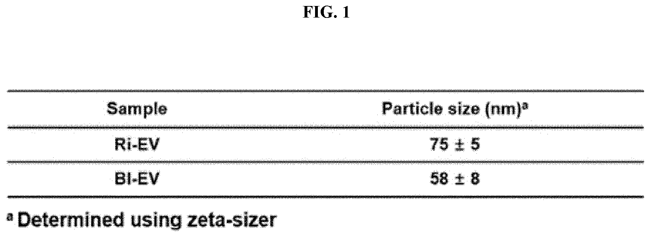

FIG. 1 shows the results of measuring the particle size of EVs derived from *Roseburia intestinalis* (Ri-EV) and EVs derived from *Bifidobacterium longum* (Bl-EV).

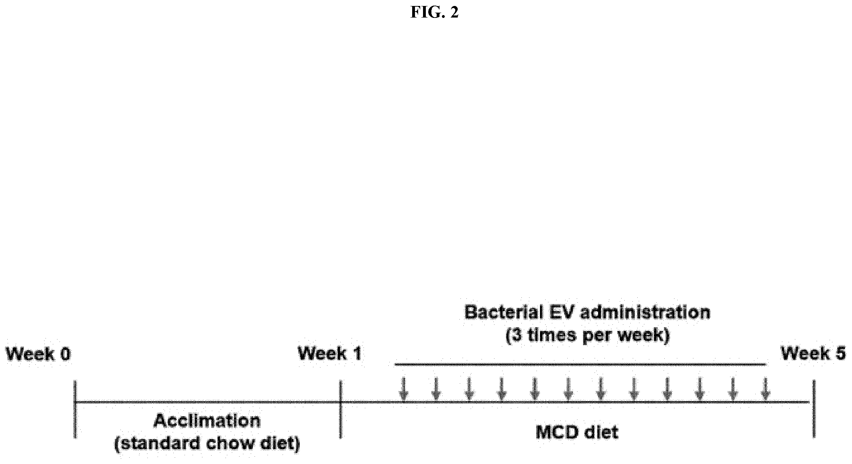

FIG. 2 shows a schematic diagram of an experiment of a NASH animal model prepared by feeding a methionine-choline deficient (MCD) diet.

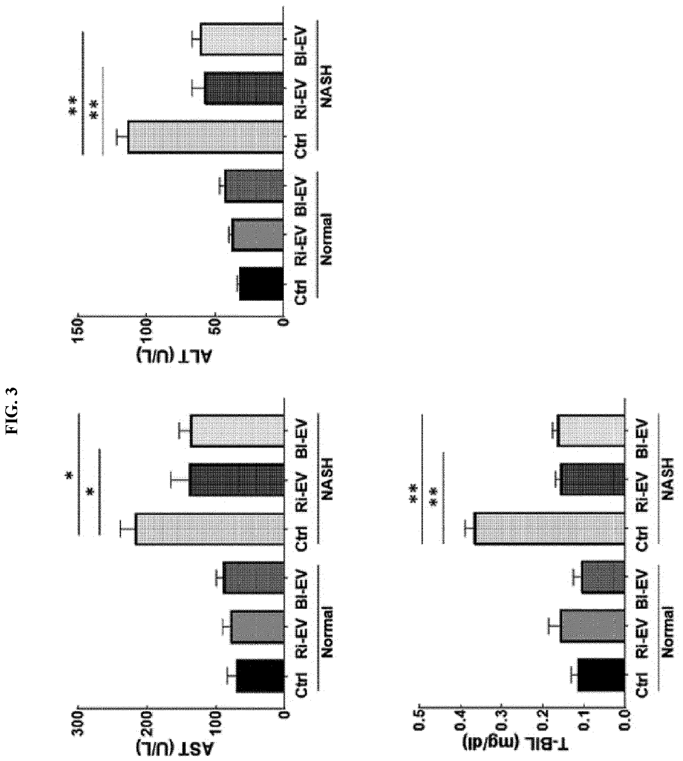

FIG. 3 shows the AST, ALT and T-BIL levels in a control group (Normal Ctrl) which was not fed an MCD diet and not administered Ri-EV or Bl-EV; experimental groups administered Ri-EV (Normal Ri-EV) or Bl-EV (Normal Bl-EV) and not fed an MCD diet; a control group fed an MCD diet and not administered Ri-EV or Bl-EV (NASH animal model, NASH Ctrl); and experimental groups fed an MCD diet and administered Ri-EV (NASH Ri-EV) or Bl-EV (NASH Bl-EV).

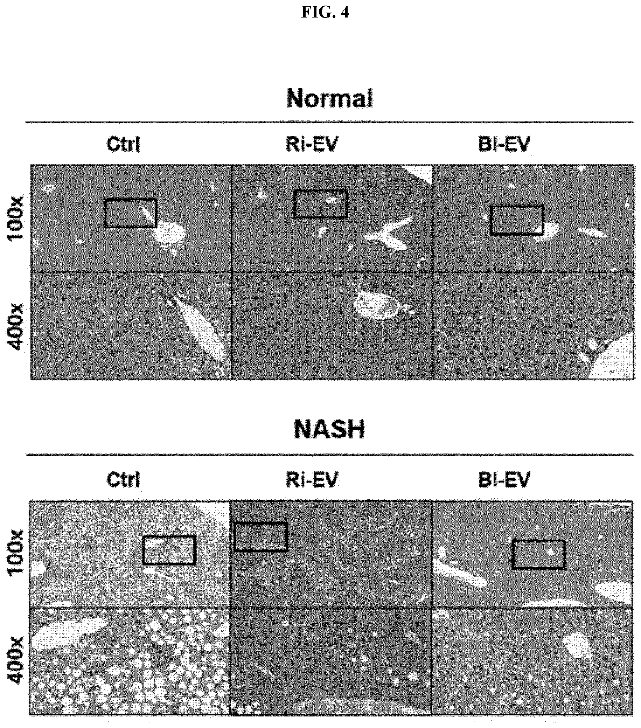

FIG. 4 shows the results of staining the liver tissue of a control group (Normal Ctrl) which was not fed an MCD diet and not administered Ri-EV or Bl-EV; experimental groups administered Ri-EV (Normal Ri-EV) and Bl-EV (Normal Bl-EV) and not fed an MCD diet; a control group fed an MCD diet and not administered Ri-EV or Bl-EV (NASH Ctrl) and experimental groups fed an MCD diet and administered Ri-EV (NASH Ri-EV) or Bl-EV (NASH Bl-EV) for histopathological analysis of the liver tissue.

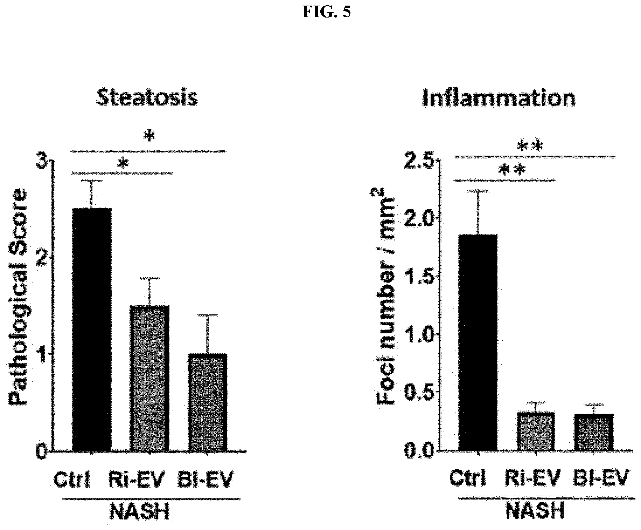

FIG. 5 shows the pathological score for steatosis and the number of inflammation-related foci of a control group fed an MCD diet and not administered Ri-EV or Bl-EV (NASH Ctrl) and experimental groups fed an MCD diet and administered Ri-EV (NASH Ri-EV) or Bl-EV (NASH Bl-EV).

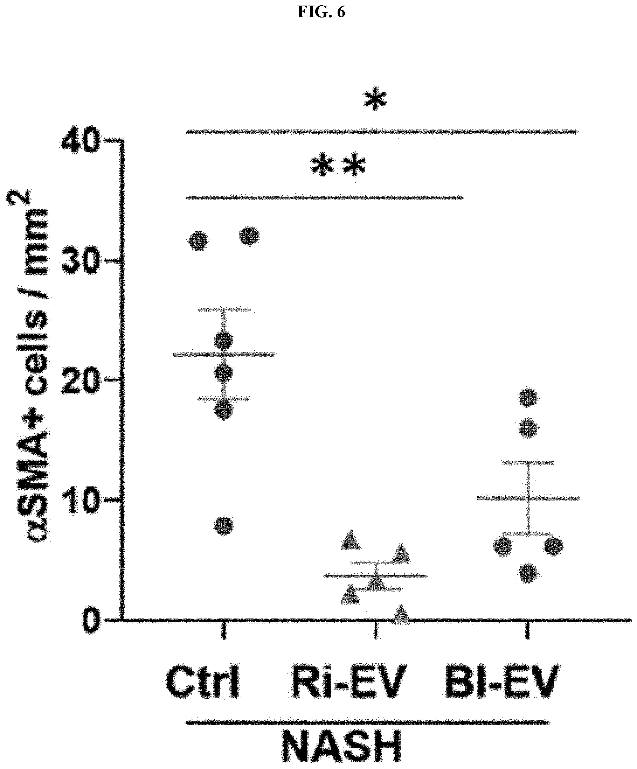

FIG. 6 shows the results of the number of myofibroblasts, measured through alpha-smooth muscle actin (α-SMA) staining, found in the liver fibrosis process of a control group fed an MCD diet and not administered Ri-EV or Bl-EV (NASH Ctrl) and experimental groups fed an MCD diet and administered Ri-EV (NASH Ri-EV) or Bl-EV (NASH Bl-EV).

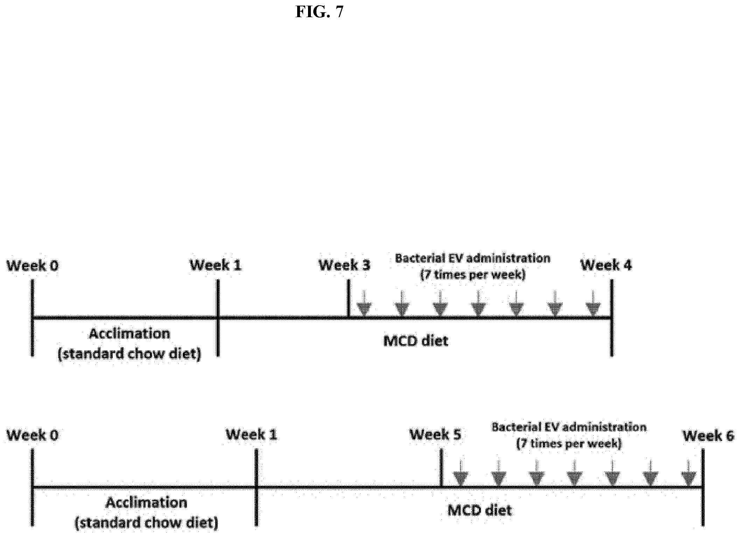

FIG. 7 shows a schematic diagram of an experiment with a NASH animal model prepared by feeding an MCD diet for two weeks (top) or 4 weeks (bottom). Ri-EV, Bl-EV, *R. intestinalis* cells (Ri-Cell), or *B. longum* cells (Bl-Cell) were each administered to the mice fed the MCT for two weeks from week 3 and to the mice fed the MCT for four weeks from week 5.

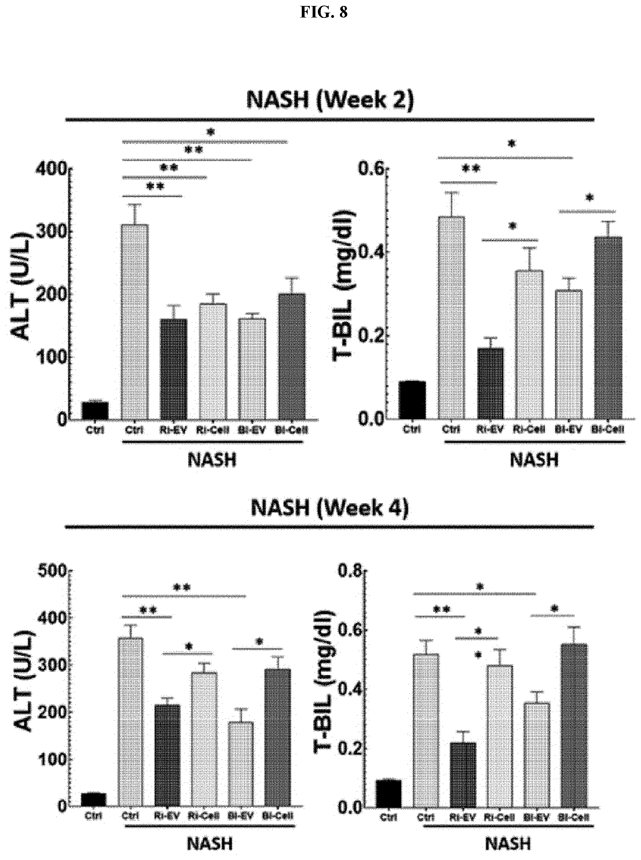

FIG. 8 shows the results of confirming the serum ALT and serum T-BIL levels of an untreated control group (Ctrl) which was not fed an MCT diet and not administered Ri-EV or Bl-EV; a NASH animal model fed an MCT diet and not administered EV or cells (NASH Ctrl); and experimental groups fed an MCT diet and administered Ri-EV (NASH Ri-EV), Ri-Cell (NASH Ri-Cell), Bl-EV (NASH Bl-EV), or Bi-Cell (NASH BI-Cell) for one week.

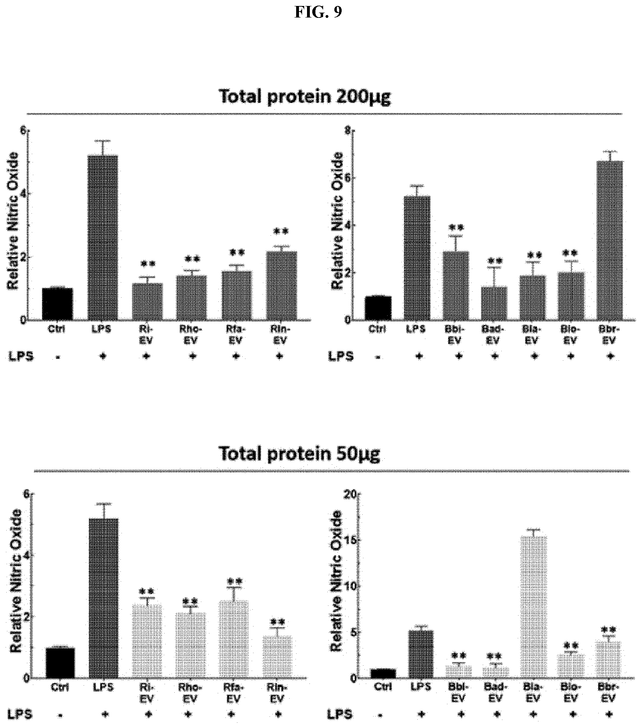

FIG. 9 shows the results of confirming the inflammation inhibitory effect through the measurement of the amount of nitric oxide production according to the treatment of EVs derived from *Roseburia spp.* strains (*R. intestinalis, R. faecis, R. hominis,* and *R. inulinivorans*) and *Bifidobacterium* spp. strains (*B. bifidum, B. breve, B. lactis, B. adolescentis,* and *B. longum*) after inducing an inflammatory environment by treating a Kupffer cell-simulated *in vitro* environment with lipopolysaccharide (LPS).

### [Modes of the Invention]

Hereinafter, the present invention will be specifically described. Meanwhile, each description and embodiment disclosed in the present invention may also be applied to each other description and embodiment. In other words, all combinations of the various elements disclosed in the present invention fall within the scope of the present invention. In addition, the scope of the present invention is not limited by the specific descriptions described below.

The present invention relates to a pharmaceutical composition for preventing or treating NAFLD, including one or more selected from the group consisting of EVs derived from a *Roseburia* spp. strain and EVs derived from a *Bifidobacterium* spp. strain as an active ingredient, and it was completed by confirming that a *Roseburia* spp. strain, a *Bifidobacterium* spp. strain, or EVs derived therefrom may ameliorate liver function and inhibit liver inflammation and fibrosis, thereby exhibiting excellent therapeutic effects in an NAFLD animal model.

The composition according to the present invention may include one or more selected from the group consisting of a *Roseburia* spp. strain, a lysate thereof, a culture solution thereof, EVs thereof, and a mixture of these; and a *Bifidobacterium* spp. strain, a lysate thereof, a culture solution thereof, EVs thereof, and a mixture of these. In one embodiment of the present invention, a composition according to the present invention may not include a strain.

In the present invention, *"Roseburia* spp. strain" or "genus *Roseburia* strain" includes strains that are already known in the art or that will be newly known in the future as strains belonging to the *Roseburia* genus. *Roseburia* is a Gram-positive bacterium inhabiting the human colon and is known to have an ability to decompose sugar and produce butyrate. Any strains belonging to the *Roseburia* genus are included as the *Roseburia* spp. strains according to the present invention without limitation, and they are not limited to specific types, but a *Roseburia* spp. strain may preferably be one or more selected from the group consisting of *Roseburia intestinalis, Roseburia faecis, Roseburia hominis, Roseburia inulinivorans,* and *Roseburia cecicola.* Among these, *"Roseburia intestinalis,"* which is a microorganism first isolated from human feces, is known to maintain energy homeostasis by producing metabolites in the intestine.

In the present invention, *"Bifidobacterium* spp. strain" or "genus *Bifidobacterium* strain" includes strains that are already known in the art or that will be newly known in the future as strains belonging to the *Bifidobacterium* genus. *Bifidobacterium* is a Gram-positive anaerobic bacterium and is one of the main types of bacteria constituting the gastrointestinal tract microflora of mammals. Some *Bifidobacteria* are also used as lactic acid bacteria. Any strains belonging to the *Bifidobacteria* genus are included as the *Bifidobacteria* spp. strains according to the present invention without limitation, and they are not limited to specific types, but a *Bifidobacteria* spp. strain may preferably be one or more selected from the group consisting of *Bifidobacterium longum, Bifidobacterium bifidum, Bifidobacterium breve, Bifidobacterium lactis,* and *Bifidobacterium adolescentis.* Among these, "*Bifidobacterium longum*" is also called Bifidobacterium and is known to be effective in inhibiting the growth of *Escherichia coli* and enhancing intestinal motility and bowel movements.

In the present invention, the term "strain" includes not only live cells themselves obtained from cultures such as culture media, but also any processed forms of strains known to those skilled in the art, for example, cell lysates, dried products, frozen products, etc., but is not limited thereto. In the present specification, the term "strain" may be used interchangeably with "bacteria" and "cells."

In the present invention, the term "lysate" may refer to a product obtained by crushing the cell wall of a strain by applying chemical treatment or physical force to the strain.

In the present invention, the term "culture solution" may refer to the entire medium including a strain, a metabolite thereof, and remaining nutrients, obtained by culturing the strain for a certain period of time in a medium capable of supplying nutrients so that the strain may grow and survive *in vitro.* In addition, the culture solution may mean a culture solution obtained by removing cells from a cell culture solution obtained by culturing a strain. Meanwhile, a liquid from which cells are removed from the culture solution is also referred to as "supernatant," and it may be obtained by allowing a culture solution to stand for a certain period of time to take only the liquid of the upper layer excluding the part that has settled in the lower layer or by taking the liquid in the upper portion after removing the cells through filtration or centrifuging the culture solution to remove the lower sediment. The "cell" refers to the strain itself of the present invention and includes the selected strain itself isolated from biological samples or the like or the strain isolated from a culture solution in which the strain has been cultured. The cells may be obtained by centrifuging a culture solution and taking the part that has settled in the lower layer, or they can be obtained by removing the liquid in the upper portion after allowing the culture solution to stand for a certain period of time because the cells have settled in the lower layer of the culture solution by gravity. The culture solution may be used interchangeably with "culture supernatant, "conditioned culture solution," or "conditioned medium."

The culture solution may include a culture medium itself obtained by culturing a strain, an extract thereof, a concentrate thereof, or a lyophilized product thereof; or a culture supernatant obtained by removing a strain from a culture solution, an extract thereof, a concentrate thereof, or a lyophilized product thereof, but is not limited thereto.

The culture solution may be obtained by culturing the strain of the present invention in an appropriate medium (e.g., reinforced clostridial medium (RCM) medium) at any temperature of 10 °C to 50 °C, 10 °C to 40 °C, 20 °C to 50 °C, 20 °C to 40 °C, or 30 °C to 40 °C for a certain period of time, for example, 4 to 50 hours, 4 to 40 hours, 4 to 30 hours, 4 to 20 hours, or 10 to 20 hours, but is not limited thereto. The culture medium and culture conditions for culturing the strain of the present invention may be appropriately selected or modified by those skilled in the art.

In one embodiment, the culture supernatant of the present invention may be obtained by removing the strain from the above-described strain culture solution by a method such as centrifugation, filtration, and the like.

In the present invention, the "concentrate" may be obtained by concentrating the above-described culture solution itself or a supernatant obtained from the culture solution by a method such as centrifugation, filtration, and the like.

In the present invention, the term "extract" refers to an extract from the above-described culture medium or a concentrate thereof and may include an extract, a diluent or concentrate of the extract, a dried or lyophilized product obtained by drying the extract, or a crude product or a purified product thereof, or a fraction obtained by fractionating the same.

In the present invention, the term "vesicle" refers to particles secreted from cells and released into the extracellular space and may include a number of different types such as exosomes, ectosomes, microvesicles, microparticles, and exosome-like vesicles. Extracellular vesicles (EVs) may reflect the state of the cells of origin (donor cells) secreting them, exhibit various biological activities depending on which cell they are secreted from, and play an important role in cell-to-cell interactions while transferring genetic materials and proteins between cells. In addition, cell-derived substances including the vesicles have the effects of causing diseases or stimulating immune cells to fight against diseases and helping to decompose and absorb substances that humans are unable to digest, through the metabolic process of microorganisms. The vesicles may be membrane-structured EVs, which are divided into an inside and an outside, have plasma membrane lipids, plasma membrane proteins, nucleic acids, and cytoplasmic components, and are smaller than the original cells. The term "EV" may be used interchangeably with "extracellular endoplasmic reticulum" and may be briefly referred to as "endoplasmic reticulum" or "vesicles."

In particular, the vesicles derived from Gram-positive bacteria such as the *Roseburia* spp. strain and *Bifidobacterium* spp. strain of the present invention may include peptidoglycan and lipoteichoic acid, which are components of the bacterial cell wall, in addition to proteins and nucleic acids.

In one embodiment of the present invention, the *Roseburia* spp. strain-derived EVs may have a single lipid membrane structure, but are not limited thereto.

Vesicles according to the present invention may be naturally secreted from a *Roseburia* spp. or *Bifidobacterium* spp. strain according to the present invention or artificially produced (isolated) through heat treatment, pressure treatment, or the like of the bacteria, and may have a diameter of 30 to 200 nm, 30 to 150 nm, 30 to 100 nm, 30 to 80 nm, 40 to 200 nm, 50 to 200 nm, 50 to 100 nm, or 50 to 80 nm. In one embodiment of the present invention, as a result of measuring the sizes of *R. intestinalis*-derived EVs and *B. longum-derived* EVs using a Zetasizer, it was confirmed that they had an average particle size of about 75 nm and 58 nm, respectively (FIG. 1).

In addition, the *Roseburia* spp. EVs according to the present invention may have a surface charge of -20 to 5 mV, -20 to 0 mV, -10 to 0 mV, -5 to 0 mV, -10 to -1 mV, or -5 to -1 mV, but are not limited thereto.

The vesicles may be isolated by applying one or more methods selected from the group consisting of centrifugation, ultra-high-speed centrifugation, extrusion, sonication, cell lysis, homogenization, freeze-thaw, electroporation, mechanical disassembly, chemical treatment, filtration with a filter, gel filtration chromatography, free-flow electrophoresis, and capillary electrophoresis to a culture solution including a *Roseburia* spp. or *Bifidobacterium* spp. strain. In addition, processes such as washing to remove impurities and concentration of the obtained vesicles may be further included.

The composition according to the present invention may have effects of preventing, ameliorating, and/or treating NAFLD.

For example, a composition according to the present invention may have one or more effects selected from among the following:
i) reducing serum AST levels or activity;
ii) reducing serum ALT levels or activity;
iii) reducing serum T-BIL levels;
iv) reducing hepatic steatosis;
v) reducing inflammatory cell infiltration;
vi) reducing the number of inflammatory foci; and
vii) reducing the number of myofibroblasts.

In other words, the effects of preventing, ameliorating, and/or treating NAFLD of the composition according to the present invention may be exerted by the above effects i) to vii).

Regarding the effects of the composition of the present invention of i) reducing serum AST levels or activity; ii) reducing serum ALT levels or activity; and iii) reducing serum T-BII, levels, the present inventors confirmed that when the EVs derived from a *Roseburia* spp. or *Bifidobacterium* spp. strain of the present invention were administered to disease-free normal mice, there was no change in serum AST, ALT, and T-BIL levels, which are indicators of liver function, whereas when the EVs were administered to an NAFLD animal model, the serum AST, ALT, and T-BIL levels were reduced compared to an untreated control group (FIG. 3). The above results show that the EVs derived from a *Roseburia* spp. or *Bifidobacterium* spp. strain of the present invention restores liver function without side effects in NAFLD.

Regarding the effects of the composition of the present invention of iv) reducing hepatic steatosis; v) reducing inflammatory cell infiltration; and vi) reducing the number of inflammatory foci, the present inventors confirmed that when EVs of the present invention were administered to normal mice, liver tissue damage and inflammatory responses were not observed, whereas when EVs derived from a *Roseburia* spp. or *Bifidobacterium* spp. strain were administered to an NAFLD animal model, steatosis and the infiltration of inflammatory cells were significantly reduced at the foci of liver tissue, and the pathological score for steatosis and the number of inflammation-related foci were significantly reduced (FIG. 5). The above results show that the EVs derived from a *Roseburia* spp. or *Bifidobacterium* spp. strain of the present invention inhibit fat accumulation in the liver and exert an anti-inflammatory effect.

Regarding the effect of the composition of the present invention of vii) reducing the number of myofibroblasts, the present inventors confirmed that when EVs derived from a *Roseburia* spp. or *Bifidobacterium* spp. strain were administered to an NAFLD animal model, the number of myofibroblasts found in the liver fibrosis process was significantly reduced (FIG. 6). The above results show that the EVs derived from a *Roseburia* spp. or *Bifidobacterium* spp. strain of the present invention may inhibit liver fibrosis caused by non-alcoholic fatty liver.

Furthermore, as a result of isolating EVs from each of various congeneric strains belonging to *Roseburia* spp. or *Bifidobacterium* spp. and confirming their anti-inflammatory effect, the present inventors confirmed that all of the EVs of the strains exert a strong anti-inflammatory effect. The above results show that the EVs derived from strains belonging to *Roseburia* spp. or *Bifidobacterium* spp. exhibit excellent anti-inflammatory effects and thus may be used as a therapeutic agent for non-alcoholic fatty liver.

In summary, the composition according to the present invention may exert anti-inflammatory and anti-fibrotic effects on the liver and improve liver function, and thus may be used for preventing, ameliorating, and/or treating non-alcoholic fatty liver.

Meanwhile, the composition according to the present invention may be used for preventing, ameliorating, and/or treating "inflammatory diseases." In the present invention, inflammatory diseases include without limitation any diseases related to inflammation or diseases that may be ameliorated/treated by inhibiting inflammation, and are not limited to specific types, but are preferably selected from the group consisting of gastritis, gastric ulcers, duodenal ulcers, inflammatory skin disease, allergic diseases, Crohn's disease, irritable bowel syndrome, ulcerative colitis, inflammatory bowel disease, peritonitis, osteomyelitis, cellulitis, meningitis, encephalitis, pancreatitis, trauma-induced shock, bronchial asthma, cystic fibrosis, stroke, acute bronchitis, chronic bronchitis, acute bronchiolitis, chronic bronchiolitis, osteoarthritis, gout, spondyloarthropathy, ankylosing spondylitis, Reiter's syndrome, psoriatic arthropathy, enteropathic spondylitis, juvenile arthropathy, juvenile ankylosing spondylitis, reactive arthropathy, infectious arthritis, post-infectious arthritis, gonococcal arthritis, tuberculous arthritis, viral arthritis, fungal arthritis, syphilitic arthritis, Lyme disease, arthritis associated with `vasculitic syndrome,' polyarteritis nodosa, hypersensitivity vasculitis, Lou Gehrig's granulomatosis, rheumatic polymyalgia, articular cell arteritis, calcium crystal deposition arthropathy, pseudogout, non-articular rheumatism, bursitis, tenosynovitis, epicondylitis (tennis elbow), neuropathic joint disease (charcot and joint), hemorrhagic arthrosis (hemarthrosis), Henoch-Schönlein purpura, hypertrophic osteoarthropathy, multicentric reticulohistiocytoma, sarcoidosis, hemochromatosis, sickle cell anemia and other hemoglobinopathies, hyperlipoproteinemia, hypogammaglobulinemia, familial Mediterranean fever, Behcet's disease, systemic lupus erythematosus, relapsing fever, psoriasis, multiple sclerosis, sepsis, septic shock, multiorgan dysfunction syndrome, acute respiratory distress syndrome, chronic obstructive pulmonary disease, acute lung injury, and broncho-pulmonary dysplasia.

In addition, the composition according to the present invention may be used for the purpose of preventing, ameliorating, and/or treating "fibrosis (fibrotic diseases)." The fibrosis is a disease in which normal tissue structures are destroyed and hardened as fibrous connective tissue is excessively formed in tissue. In the present invention, the fibrosis includes liver fibrosis, pulmonary fibrosis, skin fibrosis, pancreatic fibrosis, systemic sclerosis, and cardiac fibrosis, but is not limited to specific types.

In addition, the composition according to the present invention may be used for the purpose of preventing, ameliorating, and/or treating fatty liver disease. The fatty liver-related disease is not limited to a specific type and may include any diseases induced by excessive accumulation of fat in the liver regardless of the cause. For example, the fatty liver-related disease includes alcoholic fatty liver and non-alcoholic fatty liver.

Most preferably, the composition according to the present invention may be used for the purpose of preventing, ameliorating, and/or treating NAFLD.

In the present invention, "non-alcoholic fatty liver disease (NAFLD)" is a metabolic liver disease resulting from metabolic dysregulation, and it is a disease caused by fat accumulation in the liver that is not related to alcohol consumption. As described above, NAFLD is a group of diseases exhibiting simple steatosis with only excessive accumulation of fat in liver cells and NASH with liver cell necrosis and inflammation as well as fibrosis in which liver tissue hardens as the disease progresses. NAFLD may include any disease without limitation as long as it is a disease caused by the accumulation of fat in the liver due to causes other than alcohol, and it is not limited to specific types, but for example, it may include steatosis, fibrosis, non-alcoholic steatohepatitis (NASH), non-alcoholic fatty liver (NAFL), and cirrhosis. In addition, other liver diseases caused by the progression of NAFLD may be included in the scope of the present invention without being particularly limited. The fibrosis may be used interchangeably with fibrotic disease.

The content of the strain, lysate, culture solution, and/or EVs in the composition of the present invention may be appropriately adjusted according to the symptoms of the disease, the degree of progression of the symptoms, the conditions of the patient, etc., and for example, it may be 0.0001% to 99.9% by weight, or 0.001% to 50% by weight, but is not limited thereto. The content ratio is a value based on the dry amount after removing the solvent.

The pharmaceutical composition according to the present invention may further include appropriate carriers, excipients, and diluents that are commonly used in preparation of pharmaceutical compositions. The excipient may be, for example, one or more selected from the group consisting of diluents, binders, disintegrants, lubricants, adsorbents, humectants, film-coating materials, and controlled-release additives.

The pharmaceutical composition according to the present invention may be formulated in the form of powder, granules, sustained-release granules, enteric-coated granules, solutions, eye drops, elixirs, emulsions, suspensions, spirits, troches, perfumes, limonade, tablets, sustained-release tablets, enteric-coated tablets, sublingual tablets, hard capsules, soft capsules, sustained-release capsules, enteric-coated capsules, pills, tinctures, soft extracts, dry extracts, liquid extracts, injections, capsules, perfusates, plasters, lotions, pastes, sprays, inhalants, patches, sterilized injection solutions, or external preparations such as aerosols by conventional methods and used, and the external preparations may be formulated as creams, gel, patches, sprays, ointments, plasters, lotions, liniments, pastes, or cataplasmas.

Carriers, excipients, and diluents that may be included in the pharmaceutical composition according to the present invention include lactose, dextrose, sucrose, oligosaccharides, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

In the case of formulation, the composition according to the present invention may be prepared using diluents or excipients such as commonly used fillers, extenders, binders, wetting agents, disintegrants, and surfactants.

As additives for tablets, powder, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, calcium hydrogen phosphate, calcium sulfate, sodium chloride, sodium bicarbonate, purified lanolin, microcrystalline cellulose, dextrin, sodium alginate, methylcellulose, sodium carboxymethylcellulose, kaolin, urea, colloidal silica gel, hydroxypropyl starch, hydroxypropylmethyl cellulose (HPMC) 1928, HPMC 2208, HPMC 2906, HPMC 2910, propylene glycol, casein, calcium lactate, and Primogel; binders such as gelatin, gum arabic, ethanol, agar powder, cellulose acetate phthalate, carboxymethyl cellulose, calcium carboxymethyl cellulose, glucose, purified water, sodium caseinate, glycerin, stearic acid, sodium carboxymethyl cellulose, sodium methyl cellulose, methyl cellulose, microcrystalline cellulose, dextrin, hydroxycellulose, hydroxypropyl starch, hydroxymethyl cellulose, refined shellac, starch gelatin, hydroxypropyl cellulose, hydroxypropyl methylcellulose, polyvinyl alcohol, and polyvinylpyrrolidone may be used; disintegrants such as hydroxypropyl methyl cellulose, corn starch, agar powder, methyl cellulose, bentonite, hydroxypropyl starch, sodium carboxymethyl cellulose, sodium alginate, calcium carboxymethyl cellulose, calcium citrate, sodium lauryl sulfate, silicic acid anhydride, 1-hydroxypropyl cellulose, dextran, ion exchange resins, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, white sugar, magnesium aluminum silicate, D-sorbitol solution, and light anhydrous silicic acid; and lubricants such as calcium stearate, magnesium stearate, stearic acid, hydrogenated vegetable oils, talc, *Lycopodium* spores, kaolin, Vaseline, sodium stearate, cacao fat, sodium salicylate, magnesium salicylate, polyethylene glycol (PEG) 4000, PEG 6000, liquid paraffin, hydrogen-added soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oils, paraffin oils, polyethylene glycol fatty acid ether, starch, sodium chloride, sodium acetate, sodium oleate, dl-leucine, and light anhydrous silicic acid may be used.

As additives for liquid formulations according to the present invention, water, dilute hydrochloric acid, dilute sulfuric acid, sodium citrate, sucrose monostearate, polyoxyethylene sorbitol fatty acid esters (Tween esters), polyoxyethylene monoalkyl ethers, lanolin ethers, lanolin esters, acetic acid, hydrochloric acid, ammonia water, ammonium carbonate, potassium hydroxide, sodium hydroxide, prolamin, polyvinylpyrrolidone, ethyl cellulose, sodium carboxymethyl cellulose or the like may be used.

A solution of white sugar, other sugars, or sweeteners or the like may be used in the syrup according to the present invention, and flavoring agents, colorants, preservatives, stabilizers, suspending agents, emulsifiers, thickening agents or the like may be used as needed.

Purified water may be used in the emulsions according to the present invention, and emulsifiers, preservatives, stabilizers, flavoring agents or the like may be used as needed.

Suspending agents such as acacia, tragacanth, methylcellulose, carboxymethylcellulose, sodium carboxymethylcellulose, microcrystalline cellulose, sodium alginate, HPMC, HPMC 1828, HPMC 2906, HPMC 2910 or the like may be used in the suspensions according to the present invention, and surfactants, preservatives, stabilizers, colorants, and flavoring agents may be used as needed.

The injections according to the present invention may include solvents such as distilled water for injection, 0.9% sodium chloride for injection, Ringer's solution, dextrose for injection, dextrose + sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid, benzyl benzoate; solubilizing agents such as sodium benzoate, sodium salicylate, sodium acetate, urea, urethane, monoethyl acetamide, butazolidine, propylene glycol, Tweens, nicotinic acid amide, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediaminetetraacetic acid; antioxidants agents such as sodium bisulfide 0.1%, sodium formaldehyde sulfoxylate, thiourea, disodium ethylenediaminetetraacetate, and acetone sodium bisulfite; analgesics such as benzyl alcohol, chlorobutanol, procaine hydrochloride, glucose, and calcium gluconate; and suspending agents such as carboxymethyl cellulose (CMC) sodium, sodium alginate, Tween 80, and aluminum monostearate.

In the suppositories according to the present invention, bases such as cacao fat, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, Hexalide Base 95, Cotomar, Hydrocote SP, S-70-XXA, S-70-XX75 (S-70-XX95), Hydrokote 25, Hydrokote 711, Idropostal, Massa estrarium (A, AS, B, C, D, E, I, T), Massa-MF, Massaupol, Masupol-15, Neosupostal-N, Paramount-B, Suposiro (OSI, OSIX, A, B, C, D, H, L), suppositories base type IV (AB, B, A, BC, BBG, E, BGF, C, D, 299), Supostal (N, Es), Wecobi (W, R, S, M, Fs), Tegester triglyceride base (TG-95, MA, 57) may be used.

Solid preparations for oral administration include tablets, pills, powder, granules, and capsules. These solid preparations are prepared by mixing the extract with at least one excipient, for example, starch, calcium carbonate, sucrose, lactose, gelatin, etc. In addition to simple excipients, lubricants such as magnesium stearate and talc are also used.

Suspensions, oral solutions, emulsions, syrup, and the like correspond to liquid preparations for oral administration, and in addition to the commonly used simple diluents such as water and liquid paraffin, various excipients, for example, wetting agents, sweeteners, flavoring agents, and preservatives may be included. Preparations for parenteral administration include sterilized aqueous solutions, non-aqueous solutions, suspensions, emulsions, freeze-dried preparations, and suppositories. As non-aqueous solvents and suspensions, propylene glycol, polyethylene glycol, vegetable oils such as olive oil, and an injectable ester such as ethyl oleate may be used.

The pharmaceutical composition according to the present invention is administered in a pharmaceutically effective amount. In the present invention, "pharmaceutically effective amount" refers to an amount that is sufficient to treat a disease with a reasonable benefit/risk ratio applicable to medical treatment, and an effective dose level may be determined based on factors including the type and severity of a patient's disease, drug activity, sensitivity to the drug, administration time, administration route and excretion rate, treatment duration, and concurrently used drugs, and other factors well known in the medical field.

The pharmaceutical composition according to the present invention may be administered as an individual therapeutic agent or in combination with another therapeutic agent, and it may be administered sequentially or simultaneously with conventional therapeutic agents, and it may be administered once or multiple times. It is important to administer an amount that may achieve the maximum effect with the minimum amount without side effects by considering all of the above factors, and this may be easily determined by those skilled in the art to which the present invention pertains.

The pharmaceutical composition of the present invention may be administered to a subject through various routes. All modes of administration may be considered, and it may be administered by, for example, oral administration, subcutaneous injection, intraperitoneal administration, intravenous injection, intramuscular injection, paraspinal space (intrathecal) injection, sublingual administration, buccal administration, intrarectal injection, vaginal injection, ocular administration, auricular administration, nasal administration, inhalation, spraying through the mouth or nose, dermal administration, transdermal administration or the like.

The pharmaceutical composition of the present invention is determined according to the type of drug that is an active ingredient along with various relevant factors such as the disease to be treated, administration route, the patient's age, gender, and weight, and the severity of the disease. Specifically, the effective amount of the composition according to the present invention may vary depending on the patient's age, sex, and weight, and is generally 0.001 to 150 mg, preferably 0.01 to 100 mg per 1 kg of body weight, and may be administered daily or every other day, or 1 to 3 times a day. However, since it may increase or decrease depending on the administration route, severity of disease, sex, weight, age, etc., the dosage does not limit the scope of the present invention in any way.

In the present invention, "subject" refers to a subject in need of treatment of a disease, and more specifically, human or non-human primates, and mammals such as mice, rats, dogs, cats, horses, and cows.

In the present invention, "administration" refers to providing a predetermined amount of the composition of the present invention to a subject by any appropriate method.

In the present invention, "prevention" refers to all actions that suppress or delay the onset of a target disease, "treatment" refers to all actions that improve or beneficially change a target disease and associated metabolic abnormalities thereof by administration of the pharmaceutical composition according to the present invention, and "amelioration" refers to all actions that reduce parameters related to a target disease, for examples, the degree of symptoms, by administration of the pharmaceutical composition according to the present invention

In addition, the present invention provides a kit for preventing, ameliorating, or treating NAFLD, including the composition according to the present invention.

The kit according to the present invention is not limited to specific form as long as it may achieve the purpose of preventing, ameliorating, or treating NAFLD, and it may include without limitation any components and devices for the preparation (e.g., culturing of strains, isolation of EVs, etc.), storage, administration, etc. of the composition according to the present invention. In addition, the kit may include an instruction manual containing the general description of the composition of the present invention, such as the properties of the composition, methods of using it, methods of storing it, and the dosage.

In addition, the present invention provides a food composition for preventing or ameliorating NAFLD, including one or more selected from the group consisting of EVs derived from a *Roseburia* spp. strain and EVs derived from a *Bifidobacterium* spp. strain as an active ingredient. The food composition includes a health functional food composition.

When the strain, lysate, culture solution, and/or EVs of the present invention are used as a food additive, the strain, EVs, and the like may be added as is or used together with other food or food ingredients, and may be appropriately used according to conventional methods. The content of the active ingredient in the food composition may be appropriately determined depending on the purpose of use (prevention, amelioration or therapeutic treatment). The mixing amount of the active ingredient may be appropriately determined according to the purpose of use (prevention, health or therapeutic treatment). In general, when preparing food or beverages, the strain, EVs, and the like of the present invention may be added in an amount of 15% by weight or less, or 10% by weight or less based on the raw materials. However, in the case of long-term intake for the purpose of health and hygiene or health control, the amount may be less than he above range, and since there is no problem in terms of safety, the active ingredient may also be used in an amount greater than the above range.

There is no particular limitation on the type of food. Examples of food to which the above substances may be added include meat, sausages, bread, chocolates, candies, snacks, confectioneries, pizza, ramen, other noodles, gum, dairy products including ice cream, various soups, beverages, tea, drinks, alcoholic beverages, and vitamin complexes, and include all health functional foods in a conventional sense.

The health beverage composition according to the present invention may contain various flavoring agents or natural carbohydrates as additional ingredients, like conventional beverages. The above-described natural carbohydrates are monosaccharides such as glucose and fructose, disaccharides such as maltose and sucrose, polysaccharides such as dextrin and cyclodextrin, and sugar alcohols such as xylitol, sorbitol, and erythritol. As a sweetener, natural sweeteners such as thaumatin and stevia extract, or synthetic sweeteners such as saccharin and aspartame may be used. The proportion of the natural carbohydrates is generally about 0.01 to 0.20 g, or about 0.04 to 0.10 g per 100 mL of the composition of the present invention.

In addition to the above, the composition of the present invention may contain various nutrients, vitamins, electrolytes, flavoring agents, colorants, pectic acid and salts thereof, alginic acid and salts thereof, organic acids, protective colloidal thickeners, pH regulators, stabilizers, preservatives, glycerin, alcohols, carbonating agents used in carbonated beverages, and the like. In addition, the composition of the present invention may contain fruit flesh for preparing natural fruit juice, fruit juice beverages, and vegetable beverages. These ingredients may be used independently or in combination. The proportion of these additives is not important, but is generally selected in the range of 0.01 to 0.20 parts by weight per 100 parts by weight of the composition of the present invention.

In the present specification, "health functional food" is the same term as food for special health use (FoSHU), and refers to food with a high medicinal and healthcare effect that is processed to efficiently exhibit bioregulatory functions in addition to supplying nutrients. To obtain useful effects for preventing or ameliorating NAFLD, the food may be prepared in various forms such as tablets, capsules, powder, granules, liquids, and pills.

The health functional food of the present invention may be prepared by a method commonly used in the art and may be prepared by adding raw materials and ingredients commonly added in the art during preparation. In addition, unlike general drugs, there is an advantage in that the health functional food has no side effects that may occur when taking a drug for a long time by using food as a raw material, and it may have excellent portability.

In addition, the present invention provides a feed composition for preventing or ameliorating NAFLD, including one or more selected from the group consisting of EVs derived from a *Roseburia* spp. strain and EVs derived from a *Bifidobacterium* spp. strain as an active ingredient.

The term "feed" of the present invention refers to any natural or artificial diet, one-meal diet, or ingredients of the one-meal diet suitable for animals to eat, ingest, and digest. The feed containing the graphene nanoparticles of the present invention as an active ingredient may be prepared from various types of feed known in the art and may specifically include concentrate feed, roughage, and/or special feed.

The strain, lysate, culture solution, and/or EVs of the present invention included in the feed composition of the present invention may vary depending on the purpose and conditions of use of the feed, and for example, it may be 0.01% to 100% by weight, more specifically, 1% to 80% by weight based on the total weight of the livestock feed composition, but is not limited thereto.

When the composition is prepared as a feed additive, the composition may be prepared as a highly concentrated product of 20% to 90% or in powder or granular form. The feed additive may further include any one or more of organic acids such as citric acid, fumaric acid, adipic acid, lactic acid, and malic acid, phosphates such as sodium phosphate, potassium phosphate, acid pyrophosphate, and polyphosphate (polymeric phosphate), and natural antioxidants such as polyphenols, catechin, alpha-tocopherol, rosemary extract, vitamin C, green tea extract, licorice extract, chitosan, tannic acid, and phytic acid. When prepared as feed, the composition may be formulated in a conventional feed form and may include conventional feed ingredients together.

The feed and feed additives may further include grains such as milled or ground wheat, oat, barley, corn, and rice; vegetable protein feed such as feed based on rapeseed, soybean, and sunflower; animal protein feed such as blood meal, meat meal, bone meal, and fish meal; sugars and dairy products such as dry ingredients composed of various types of powdered milk and whey powder, and they may further include nutritional supplements, digestion and absorption enhancers, growth promoters, and the like.

The feed additive may be administered to animals independently or in combination with other feed additives in an edible carrier. In addition, the feed additives may be easily administered to animals as a top dressing, after being directly mixed with animal feed, or in an oral formulation separate from feed. When the feed additive is administered separately from animal feed, it may be prepared as an immediate release or sustained release formulation by combining it with a pharmaceutically acceptable edible carrier, as is well known in the art. Such edible carriers may be solid or liquid, and they may be, for example, corn starch, lactose, sucrose, soybean flakes, peanut oil, olive oil, sesame oil, and propylene glycol. When a solid carrier is used, the feed additive may be tablets, capsules, powder, troches or sugar-containing tablets or a top dressing in a microdispersible form. When a liquid carrier is used, the feed additive may be in the form of gelatin soft capsules, or syrups, suspensions, emulsions, or solutions.

In addition, the feed and feed additives may contain auxiliary agents, such as preservatives, stabilizers, wetting agents or emulsifying agents, solution accelerators, and the like. The feed additive may be used by adding it to an animal's feed by steeping, spraying or mixing.

The feed or feed additive of the present invention may be applied to the diet of a number of animals including mammals, poultry, and fish.

As the mammals, it may be used not only for pigs, cows, horses, sheep, rabbits, goats, rodents, and laboratory rodents such as rats, hamsters, and guinea pigs, but also for companion animals (e.g., dogs and cats). As the poultry, it may also be used for chickens, turkeys, ducks, geese, pheasants, and quails, and as the fish, it may be used for carp, crucian carp, and trout, but is not limited thereto.

Hereinafter, examples will be described in detail to aid understanding of the present invention. However, the following examples are merely illustrative of the contents of the present invention, and the scope of the present invention is not limited to the following examples. The examples of the present invention are provided to more completely explain the present invention to those skilled in the art.

### [Examples]

### Example 1. Extraction and size analysis of EVs derived from Roseburia intestinalis or Bifidobacterium longum (Ri-EV, Bl-EV)

To extract EVs from each of *R. intestinalis* and *B. longum, R. intestinalis* KCTC 15746 and *B. longum* KCTC 3249 were cultured for 18 hours in an anaerobic chamber at 37 °C using reinforced clostridial medium (RCM) (MBcell, MB-R1602). After centrifuging the culture solution at 2000×g for 20 minutes, the supernatant was collected, and cell debris and waste products in the culture medium were removed using a 0.22 µm filter. EVs were extracted from the culture solution through a 300 kDa molecular weight Tangential Flow Filtration (TFF) membrane filter-based TFF system, diluted in phosphate-buffered saline (PBS), and purified, and finally *R. intestinalis-derived* EVs (Ri-EV) and *B. longum*-derived EVs (Bl-EV) dispersed in PBS were extracted.

As a result of measuring the sizes of Ri-EV and Bl-EV using a Zetasizer, it was confirmed that they had an average particle size of 75 nm and 58 nm, respectively (FIG. 1).

### Example 2. Evaluation of liver function restoration effect in NASH animal model according to EV administration

Since NAFLD is a group of diseases exhibiting simple steatosis with only excessive accumulation of fat in liver cells and NASH with liver cell necrosis, inflammation, and fibrosis as the disease progresses (Brunt EM, 2001), a NASH animal model was used to evaluate whether liver function was restored by the administration of Ri-EV and Bl-EV extracted in Example 1. The process of producing the NASH animal model is shown in FIG. 2.

A NASH animal model was produced by feeding C57BL/6 mice a methionine-choline deficient (MCD) diet for four weeks. The experimental groups were orally administered Ri-EV or Bl-EV three times a week for four weeks while being fed an MCD diet based on 200 µg/mouse/day of total protein. These groups were compared with a control group (NASH animal model) fed an MCD diet without being administered Ri-EV or Bl-EV.

In addition, normal mice not fed an MCD diet were also orally administered Ri-EV or Bl-EV three times a week for four weeks based on 200 µg/mouse/day of total protein, and these groups were compared with a control group which was not fed MCD and not administered Ri-EV or Bl-EV.

For blood biochemical analysis to evaluate liver function, mice were sacrificed four weeks after the start of the experiment, and serum was obtained. The blood biochemical analysis was performed by requesting T&P Bio to perform a liver function test. The activity of serum AST and ALT was measured using the Reitman-Frankel colorimetric kit (Asan Pharmaceutical, Korea) according to the manufacturer's procedure, and serum T-BIL was quantified using the Jendrassik-Grof enzymatic assay kit (BioAssay Systems, Hayward, CA, USA) according to the manufacturer's procedure.

As a result, as shown in FIG. 3, when Ri-EV or Bl-EV was administered to normal mice which were not fed an MCD diet, there was no change in serum AST, ALT, and T-BII, levels, which are liver function indicators, compared to the control group which was not fed an MCD diet and not administered Ri-EV or Bl-EV. On the other hand, when Ri-EV or Bl-EV was administered to the NASH animal model, the serum AST, ALT, and T-BIL levels were significantly reduced compared to the NASH animal model which was not administered EV. Through this, it was confirmed that Ri-EV administration and Bl-EV administration reduced serum AST, ALT, and T-BIL levels in a NASH animal model without side effects, thereby restoring liver function.

### Example 3. Histological evaluation of liver tissue according to EV administration in NASH animal model

For histopathological analysis of liver tissue by the administration of Ri-EV and Bl-EV extracted from Example 1, the liver was excised from the NASH animal model of Example 2, and the tissue was stained. Specifically, after the excised liver was fixed in 4% neutral buffered formalin and embedded in paraffin, 4 µm-thick tissue sections were prepared, stained with Hematoxylin-Eosin (H&E) and alpha-smooth muscle actin (α-SMA) to detect myofibroblasts found in the liver fibrosis process, and the sections were observed under an optical microscope.

As a result, as shown in FIG. 4, when Ri-EV or Bl-EV was administered to normal mice which were not fed an MCD diet, no liver tissue damage or inflammatory response was observed. On the other hand, when Ri-EV or Bl-EV was administered to the NASH animal model, it was confirmed that steatosis and infiltration of inflammatory cells were significantly reduced at the lesion site of liver tissue.

In addition, as shown in FIG. 5, the pathological score for steatosis and the number of inflammation-related foci were significantly reduced when Ri-EV or Bl-EV was administered to the NASH animal model, and as shown in FIG. 6, when Ri-EV or Bl-EV was administered to the NASH animal model, the number of myofibroblasts found in the liver fibrosis process was significantly reduced.

Through this, it was confirmed that the administration of Ri-EV and Bl-EV reduces steatosis and inflammatory cell infiltration in a NASH animal model without side effects, reduces the pathological score for steatosis and the number of inflammation-related foci, and inhibits the expression of myofibroblasts related to liver fibrosis, thereby suppressing the deterioration of liver function. In other words, the above results show that the Ri-EV and Bl-EV of the present invention may effectively ameliorate inflammation, fat accumulation, and fibrosis in the liver caused by NASH.

### Example 4. Evaluation of NASH treatment efficacy of strains and EVs in NASH animal models

As the preventive effects of Ri-EV and Bl-EV on NASH were confirmed through Example 2, in the present Example, the NASH treatment efficacy of the *Roseburia* spp. and *Bifidobacterium* spp. strains themselves (i.e., cells) and the EVs derived therefrom were compared. To this end, NASH animal models were prepared by feeding an MCD diet to C57BL/6 mice for two weeks and four weeks, respectively, according to the procedure shown in FIG. 7. As experimental groups, the mice fed an MCD diet for two weeks and four weeks were orally administered Ri-EV, Bl-EV, Ri-Cell (*R. intestinalis* cells), or Bl-Cell (*B. longum* cells) once a day at 200 µg/mouse/day based on the total protein amount for a total of one week from week 3 and week 5, respectively. These groups were compared with a control group (NASH animal model) which was fed an MCD diet and not administered a sample group (cells or EVs).

For blood biochemical analysis to evaluate liver function, mice were sacrificed one week after the start of the administration of a sample group, and the blood biochemical analysis was performed by requesting T&P Bio to perform the same.

As a result, as shown in FIG. 8, it was confirmed that in the case of the NASH animal model produced by being fed an MCD diet for two weeks, the ALT level was significantly reduced in the Ri-EV, Ri-Cell, Bl-EV, and Bl-Cell administration groups compared to the control group which was not administered the sample group (cells or EVs), and it was confirmed that the degree of reduction in the ALT level was greater in the sample groups other than Bl-Cell. It was confirmed that the T-BIL was significantly reduced in the Ri-EV and Bl-EV administration groups compared to the control group which was not administered the sample group, and in particular, this reduction was greater than that of each administration group (Ri-Cell or Bl-Cell administration group). I was confirmed that the degree of reduction in the T-BIL level relative to that of the control group was significantly greater in the Ri-EV administration group than in the Bl-Ev administration group.

In the case of the NASH animal model produced by being fed an MCD diet for four weeks, the cell administration groups showed no difference in ALT and T-BIL levels compared to the control group which was not administered a sample group, but the ALT and T-BIL levels were significantly reduced in the Ri-EV administration group or BL-EV administration group.

The above results show that the administration of the Ri-EV and BL-EV of the present invention may restore liver function by reducing ALT and T-BII, levels in a NASH animal model, and such NASH treatment is much more effective than direct administration of the cells of the EVs.

### Example 5. Analysis of anti-inflammatory activity of EVs derived from Roseburia spp. strains and Bifidobacterium spp. strains

In the present example, it was confirmed whether EVs of other strains of *Roseburia* spp. and *Bifidobacterium* spp. could exert a treatment effect on NASH, in addition to the EVs derived from *R. intestinalis* and *B. longum* extracted through the process of Example 1.

Specifically, EVs were extracted from each of four strains of *Roseburia* spp. (*Roseburia faecis,* KCTC 15739), *Roseburia hominis* (KCTC 5845), *Roseburia inulinivorans* (DSM 16841)) and each of five strains of the *Bifidobacterium* spp. (*Bifidobacterium bifidum* (KCTC 3281), *Bifidobacterium breve* (KCTC 3220), *Bifidobacterium lactis* (KCTC 5854), *Bifidobacterium adolescentis* (KCTC 3216)), and their anti-inflammatory activity was analyzed. The EVs were extracted in the same manner as in Example 1.

To evaluate the anti-inflammatory activity of the EVs from each strain in NAFLD, an *in vitro* environment simulating Kupffer cells, one type of cells constituting liver tissue, was produced using mouse macrophages Raw264.7 cells and the results of measuring the amount of nitrogen oxide (NO) produced by them are shown in FIG. 9. Specifically, mouse macrophage Raw 264.7 cells were cultured at 37 °C in the presence of 5% CO₂ in an RPMI1640 culture solution containing 10% FBS and 1% antibiotics (100 U/ml penicillin and 100 µg/ml streptomycin). Thereafter, 1 ml of the culture solution was dispensed into a 12-well plate at a concentration of 5×10⁵ cells/well and cultured at 37 °C for 24 hours in a CO₂ incubator. To induce inflammation, the wells were treated with a medium supplemented with 10 µg/ml of LPS, and culture was performed for four additional hours. Then, the vesicles were added to the medium at a concentration of 200 µg based on the total protein amount, and culture was performed at 37 °C for 16 hours. Thereafter, 50 µl of the well supernatant and 50 µl of the Griess reagent were mixed and allowed to react at room temperature for ten minutes, and then absorbance was measured at 540 nm using a plate reader to confirm the amount of NO production.

As a result, as shown in FIG. 9, it was confirmed that NO production by LPS was significantly and effectively reduced in all groups administered the EVs derived from the *Roseburia* spp. strains used as experimental groups. It was confirmed that NO production was effectively reduced also in the groups administered the EVs derived from the *Bifidobacterium* spp. strains, and among them, the ability of Bbr-EV and Bla-EV to inhibit NO production was controlled at a total protein amount of 200 µg or 50 µg.

The above results show that EVs of congeneric strains belonging to the *Roseburia* spp. or *Bifidobacterium* spp. exert excellent anti-inflammatory effects. In other words, from the above results, it was confirmed that EVs derived from *Roseburia* spp. and *Bifidobacterium* spp. strains may be used for preventing, ameliorating, or treating NAFLD through their strong anti-inflammatory effects.

As described in the above examples, it was confirmed that the EVs derived from a *Roseburia* spp. strain and a *Bifidobacterium* spp. strain of the present invention restore liver function, ameliorate steatosis by reducing fat accumulation in the liver, and exhibit anti-inflammatory effects such as inhibiting the infiltration of inflammatory cells into liver tissue, as well as inhibiting liver fibrosis by reducing the expression of myofibroblasts in a NASH animal model. Furthermore, it was found that all EVs derived from the congeneric strains belonging to *Roseburia* spp. and *Bifidobacterium* spp. strains exert strong anti-inflammatory effects. As such, the EVs derived from *Roseburia* spp. strains and EVs derived from *Bifidobacterium* spp. strains can ameliorate liver damage and dysfunction caused by fatty liver and exert strong anti-inflammatory and anti-fibrotic effects, and thus can be used as a composition for preventing, ameliorating, and/or treating NAFLD.

From the above description, those skilled in the art to which the present invention pertains will be able to understand that the present invention may be implemented in other specific forms without changing its technical spirit or essential features. In this regard, it should be understood that the above-described embodiments are illustrative in all respects and not restrictive. The scope of the present invention should be construed as including all changes or modifications derived from the meaning and scope of the claims to be described below and equivalent concepts thereof, rather than the above detailed description.

### [Industrial Applicability]

It was confirmed that the EVs derived from a *Roseburia* spp. strain and a *Bifidobacterium* spp. strain of the present invention restore liver function, ameliorate steatosis by reducing fat accumulation in the liver, and exhibit anti-inflammatory effects such as inhibiting the infiltration of inflammatory cells into liver tissue, as well as inhibiting liver fibrosis by reducing the expression of myofibroblasts in an NAFLD animal model. Furthermore, it was found that all EVs derived from the congeneric strains belonging to *Roseburia* spp. and *Bifidobacterium* spp. strains exert strong anti-inflammatory effects. As such, the EVs derived from *Roseburia* spp. strains and EVs derived from *Bifidobacterium* spp. strains can ameliorate liver damage and dysfunction caused by fatty liver and exert strong anti-inflammatory and anti-fibrotic effects, and thus can be used as a composition for preventing, ameliorating, and/or treating NAFLD.

## Claims

1. A pharmaceutical composition for preventing or treating non-alcoholic fatty liver disease (NAFLD), comprising one or more selected from the group consisting of extracellular vesicles (EVs) derived from a *Roseburia* spp. strain and EVs derived from a *Bifidobacterium* spp. strain as an active ingredient.

2. The pharmaceutical composition of claim 1, wherein the EVs have an average diameter of 30 nm to 200 nm.

3. The pharmaceutical composition of claim 1, wherein the EVs derived from a *Roseburia* spp. strain are naturally or artificially secreted from a *Roseburia* spp. strain; and the EVs derived from a *Bifidobacterium* spp. strain are naturally or artificially secreted from a *Bifidobacterium* spp. strain.

4. The pharmaceutical composition of claim 1, wherein the *Roseburia* spp. strain is one or more selected from the group consisting of *Roseburia intestinalis, Roseburia faecis, Roseburia hominis,* and *Roseburia inulinivorans.*

5. The pharmaceutical composition of claim 1, wherein the *Bifidobacterium* spp. strain is one or more selected from the group consisting of *Bifidobacterium longum, Bifidobacterium bifidum, Bifidobacterium breve, Bifidobacterium lactis,* and *Bifidobacterium adolescentis.*

6. The pharmaceutical composition of claim 1, wherein the NAFLD is one or more selected from the group consisting of steatosis, fibrosis, non-alcoholic steatohepatitis, non-alcoholic fatty liver (NAFL), and cirrhosis.

7. The pharmaceutical composition of claim 1, **characterized in that** the pharmaceutical composition has one or more effects selected from the group consisting of:
i) reducing serum aspartate aminotransferase (AST) levels or activity;
ii) reducing serum alanine aminotransferase (ALT) levels or activity;
iii) reducing serum total bilirubin (T-BIL) levels;
iv) reducing hepatic steatosis;
v) reducing inflammatory cell infiltration;
vi) reducing the number of inflammatory foci; and
vii) reducing the number of myofibroblasts.

8. A kit for preventing or treating NAFLD, comprising the pharmaceutical composition of any one of claims 1 to 7.

9. A method of preventing or treating NAFLD, comprising administering one or more selected from the group consisting of EVs derived from a *Roseburia* spp. strain and EVs derived from a *Bifidobacterium* spp. strain to a subject in need thereof.

10. A food composition for preventing or ameliorating NAFLD, comprising one or more selected from the group consisting of EVs derived from a *Roseburia* spp. strain and EVs derived from a *Bifidobacterium* spp. strain as an active ingredient.

11. The food composition of claim 10, wherein the food composition is a health functional food composition.

12. A feed composition for preventing or ameliorating NAFLD, comprising one or more selected from the group consisting of EVs derived from a *Roseburia* spp. strain and EVs derived from a *Bifidobacterium* spp. strain as an active ingredient.

13. A use of one or more selected from the group consisting of EVs derived from a *Roseburia* spp. strain and EVs derived from a *Bifidobacterium* spp. strain for preventing or treating NAFLD.

14. A use of one or more selected from the group consisting of EVs derived from a *Roseburia* spp. strain and EVs derived from a *Bifidobacterium* spp. strain for preparing a therapeutic agent for NAFLD.
